# EUROPÄISCHE PATENTSCHRIFT

(11) **EP 3 485 407 B1**
(45) Veröffentlichungstag und Bekanntmachung des Hinweises auf die Patenterteilung: **18.01.2023**
(21) Anmeldenummer: 17739990.4
(22) Anmeldetag: 14.07.2017
(51) Int. Cl.: G16H 40/63

(54) **VERFAHREN ZUM AUSTAUSCH VON DATEN BEI EINEM MEDIZINTECHNISCHEN MESSSYSTEM MIT AUSTAUSCHBAREM EINMALARTIKEL**
METHOD FOR INTERCHANGING DATA FOR A MEDICAL MEASUREMENT SYSTEM WITH A REPLACEABLE DISPOSABLE ITEM
PROCÉDÉ D'ÉCHANGE DE DONNÉES DANS UN SYSTÈME DE MESURE À USAGE MÉDICAL À ARTICLE À USAGE UNIQUE ÉCHANGEABLE

(30) Priorität: 15.07.2016 DE 102016113041
(43) Veröffentlichungstag der Anmeldung: 22.05.2019
(73) Patentinhaber: B. Braun Melsungen AG, 34212 Melsungen (DE)
(72) Erfinder: SCHMID, Günter, 82110 Germering (DE); GERSTMANN, Reinhard, 86916 Kaufering (DE)
(74) Vertreter: Winter, Brandl - Partnerschaft mbB
(86) Internationale Anmeldenummer: PCT/EP2017/067896
(87) Internationale Veröffentlichungsnummer: WO 2018/011409

(56) Entgegenhaltungen:
- WO-A1-2005/040793
- WO-A1-2009/120231
- US-A1- 2014 203 915
- Mikko Lehtonon ET AL: "From Identification to Authentication - A Review of RFID Product Authentication Techniques", Networked RFID Systems and Lightweight cryptography, 12. September 2007 (2007-09-12), XP055071500, ISBN: 978-3-64-209079-0 Gefunden im Internet: URL:http://www.im.ethz.ch/publications/RFI Dsec06.pdf [gefunden am 2013-07-16]

## Beschreibung

Die vorliegende Erfindung betrifft ein Verfahren zum Austausch von Daten eines Einmalartikels mit einem wiederverwendbaren Teil eines medizintechnischen (physiologischen) Messsystems bei einer Messung eines Blutwerts.

Im Gebiet der physiologischen Messtechnik, insbesondere zur Messung von Blutparametern, wie zum Beispiel Blutglukose, sind Messsysteme auf fluoreszenzoptischer Basis bekannt. Bei diesen Systemen findet eine Messung der Blutglukosekonzentration durch ein externes Gerät statt, beispielsweise in Form eines Point-of-Care Messgeräts, eines Blutgasanalyzers oder eines Laboranalyzers.

Um sensorspezifische Daten für das Messsystem zur Verfügung zu stellen, existieren unterschiedliche Möglichkeiten mit jeweils spezifischen Vor- und Nachteilen. Zum Beispiel kann ein sensorspezifischer, vom Menschen lesbarer Code am Einmalartikel oder an dessen Verpackung angebracht sein, der durch den Anwender am Messsystem einzugeben ist. Eine andere Möglichkeit besteht darin, optische Codes (zum Beispiel Barcodes oder Matrix-Codes) am Einmalartikel oder an dessen Verpackung anzubringen und diese Codes durch das Messsystem einzulesen. Schließlich kann der Einmalartikel mit einem elektronisch lesbaren Speicherchip versehen sein, der automatisch durch das Messsystem ausgelesen wird. Dabei stellt eine Verwendung von RFID-Technik (Radio Frequency Identification) eine besonders vorteilhafte Möglichkeit dar, sensorspezifische Daten für das Messsystem automatisiert zur Verfügung zu stellen. Dazu enthält der Einmalartikel einen RFID-Tag mit einem elektronischen Speicherchip und der wiederverwendbare Teil einen RFID-Reader/Writer.

Eine Verwendung von RFID-Technologie im Bereich der medizinischen Messtechnik ist bekannt. Insbesondere im Zusammenhang mit Sensorkassetten, die in Analysatoren eingesetzt werden und zur Identifizierung und Nachverfolgung von Probenbehältern in klinischen Analysatoren dienen, werden hierfür an den Kassetten bzw. Behältern befestigte Speicherchips eingesetzt, in welchen unterschiedliche Parameter des Sensorsystems und/oder der Proben abgelegt sind. Die DE 10 2005 052 752 A1 offenbart eine Reaktionskartusche mit einem rein optischen System, wobei ein Identifikationssystem in Form eines Barcodes oder eine Datamatrix an der Reaktionskartusche befestigt ist und mit Hilfe einer Leseoptik ausgelesen wird. Die WO 2009/062940 A1 offenbart eine Verwendung eines elektronischen Speicherelements, beispielsweise in Form eines Memorychips, einer Speicherkarte, eines RFID-Transponders oder eines Magnetstreifens, wobei das Speicherelement auf einer in einen Analysator einsetzbaren Sensorkassette angeordnet ist. Die WO 2010/087764 A1 offenbart Einweg-Steckverbinder, die jeweils mit RFID-Tags ausgerüstet sind. Diese weisen spezielle strahlungsresistente CMOS Schaltkreise auf und sind mit einem FRAM-Speicher ausgestattet. Mit einem entsprechenden Lesegerät können der Sterilisierungsgrad der Steckverbindung und der Gebrauchszustand festgestellt werden. Des Weiteren ist feststellbar, ob die Steckverbindung zulässig ist.

WO2009/120231 A1, welches als nächster Stand der Technik angesehen wird, offenbart ein Verfahren zur Erkennung einer nicht autorisierten Nutzung eines Einmalartikels mittels eines RFID-Tags, der in den Einmalartikel integriert wird und mit einem Steuermodul verbunden ist, um einen nicht erlaubten Betrieb des Einmalartikels zu verhindern. Auf Seite 12 wird offenbart, dass die Daten redundant geschrieben werden, um Datenverlust vorzubeugen.

US 2014/0203915 A1 beschreibt verbesserte Fähigkeiten für eine eigenständige RFID-Sensorvorrichtung, welche einen passiven RFID-Tag hat und derart konfiguriert ist, um Sensorinformationen von einem Sensor zu lesen. In den Absätzen [0284]-[0288] beschreibt dieses Dokument eine redundante Datenspeicherung auf einem RFID-tag.

WO2005/040793 A1 betrifft ein Messsystem, wobei ein Sensorvorrat mit einem zugeordneten Speicher, z.B. RFID, und ein Messgerät Daten, beispielsweise Identifikationsdaten, Informationen über den aktuellen Sensorvorrat oder Prüfsummen zur Bestätigung der gespeicherten Daten, austauscht. Insbesondere Absatz [0060] beschreibt eine redundante Datenhaltung um einem Schaden am dem RFID-Tag vorzubeugen.

Keines der Dokumente benennt Details bei Beschreiben des RFID-Tags und insbesondere die Verwendung einer ersten Version im Falle einer negativen Plausibilitätsprüfung unter Verwendung einer Prüfsumme.

Es ist von Nachteil, dass bekannte Systeme und Verfahren nur Teillösungen für Einzelprobleme darstellen, jedoch keinen vollständigen geschlossenen Ablauf, um einem Messsystem automatisiert Daten eines sterilisierbaren, abnehmbaren Einmalartikels zur Verfügung zu stellen und diese Daten während eines Betriebs zu modifizieren. Ein weiterer Nachteil ist, dass der Speicherinhalt eines jederzeit im Betrieb vom Anwender abnehmbaren Einmalartikels nicht derart gegen unerkannte Verfälschung gesichert ist, dass ein Abnehmen des Einmalartikels vom Messsystem durch den Anwender nicht zu korrumpierten Daten führt, die einen späteren Weiterbetrieb verhindern.

Vor diesem Hintergrund liegt der vorliegenden Erfindung die Aufgabe zugrunde, ein Verfahren zum Austausch von Daten eines Einmalartikels mit einem wiederverwendbaren Teil eines medizintechnischen Messsystems zu schaffen, das die vorgenannten Nachteile bekannter Verfahren nicht aufweist. Das Verfahren soll nutzerfreundlich sein, wobei insbesondere möglich sein soll, einen Einmalartikel zur Speicherung (sensor-)spezifischer Daten automatisiert auszulesen und zu beschreiben, den Speicherinhalt bei einer Strahlensterilisation des Einmalartikels nicht zu korrumpieren, während der Anwendung des Einmalartikels den Speicher automatisiert durch das Messsystem auszulesen, um eine Plausibilitätsprüfung des Einmalartikels durchführen zu können, während der Anwendung des Einmalartikels den Speicher automatisiert durch das Messsystem mit modifizierten Daten zu beschreiben, um eine Überwachung des Einmalartikel durchführen zu können und/oder während der Anwendung des Einmalartikels Daten aus dem Speicher für die Parametrierung von Softwarealgorithmen (z.B. Messalgorithmen) zu verwenden und den Speicherinhalt so gegen unerkannte Verfälschung sichern, dass ein jederzeitiges Abnehmen des Einmalartikels vom Messsystem durch den Anwender nicht zu derart korrumpierten Daten führt, dass ein späterer Weiterbetrieb verhindert wird.

Diese Aufgabe(n) wird (werden) nach der vorliegenden Erfindung gelöst durch ein Verfahren zum Austausch von Daten (Parametern) eines Einmalartikels z.B. Sensors mit einem wiederverwendbaren Teil eines medizintechnischen Messsystems, insbesondere eines physiologischen Messsystems, bei einer Messung eines Blutwerts, insbesondere eines Blutglukosewerts, wobei die zum Austausch vorgesehenen Daten bei einer Herstellung des Einmalartikels in einen Speicher eines RFID-Tags des Einmalartikels geschrieben werden und zumindest eine Prüfsumme nach aus dem Stand der Technik bekannter Weise aufweisen bzw. zugeordnet bekommen, der Einmalartikel mit dem Messsystem verbunden wird, insbesondere datentechnisch und körperlich verbunden wird, die im RFID-Tag enthaltenen Daten durch das Messsystem ausgelesen werden und einer Plausibilitätsprüfung anhand der zumindest einen Prüfsumme unterzogen werden und bei positiver Plausibilitätsprüfung der Einmalartikel zur Verwendung mit dem Messsystem freigegeben wird, und die Daten nach dem Prinzip der iterativen, doppelten Speicherung mit Prüfsumme auf dem RFID-Tag abgespeichert werden, bei welchem die Daten in Form eines ersten Datensatzes und eines zweiten Datensatzes auf dem RFID-Tag gespeichert werden und jeder der beiden Datensätze mit einer eigenen Prüfsummer versehen wird, wobei der erste Datensatz Daten der ersten Version und der zweite Datensatz überarbeitete Daten hat, wobei im Fall einer unvollständigen Datenüberschreibung die resultierende Prüfsumme nicht mit der theoretisch zu erwartenden Prüfsumme übereinstimmt und das wiederverwendbare Teil auf den ersten Datensatz zurückgreift.

Die vorliegende Erfindung kann insbesondere auf dem Gebiet der physiologischen Messtechnik liegen und beispielsweise konkret die Messung von Blutparametern, wie zum Beispiel Blutglukose, mit Hilfe eines Messsystems auf fluoreszenzoptischer Basis betreffen. Ein solches Messsystem kann Bestandteil einer Weiterentwicklung des vorstehend beschriebenen Systems zur Regelung der Blutglukosekonzentration von Intensivpatienten sein.

Das Messsystem umfasst einen Einmalartikel sowie einen wiederverwendbaren Teil. Der Einmalartikel des Messsystems kann zum Beispiel ein Sensor sein, der von einem Fluid wie Blut oder Spüllösung durchströmt wird und mit diesem Fluid in Kontakt stehende Fluorophore enthält. Der wiederverwendbare Teil des Messsystems enthält optoelektronische und elektronische Elemente und ist mit dem Einmalartikel lösbar (datenübertragungstechnisch und ggf. mechanisch) zu verbinden. Der Einmalartikel erhält im Rahmen seiner Assemblierung den RFID-Tag, der beispielsweise in einem Gehäuse des Einmalartikels montiert werden oder sein kann.

Vor einer Verwendung des Einmalartikels muss dieser zunächst mit dem dazu bestimmten wiederverwendbaren Teil des Messsystems (datenübertragungstechnisch und ggf. mechanisch) verbunden werden. Dies kann besonders einfach durch eine (elektrisch-mechanische) Steckverbindung der genannten Einheiten erfolgen. Im Rahmen des erfindungsgemäßen Verfahrens kann eine Software eines Steuermoduls des Messsystems (insbesondere dessen wiederverwendbaren Teils) mit Hilfe eines RFID-Readers/Writers den Speicherinhalt des RFID-Tags auslesen, sobald die Verbindung zwischen Einmalartikel und Messsystem erstellt ist. Sodann kann die Software eine oder mehrere Plausibilitätsprüfungen hinsichtlich des Speicherinhalts des RFID-Tags durchführen. Diese können nach der Erfindung insbesondere eine Prüfung auf korrekte Version und/oder eine Prüfung auf korrekte Prüfsummen und/oder eine Prüfung auf gültige applikationsspezifische Kenndaten und/oder eine Prüfung auf chargenspezifische Kenndaten umfassen (zum Beispiel dass eine maximale Applikationsdauer nicht überschritten ist). Bei positivem Prüfergebnis kann die Software den Einmalartikel zur Verwendung freigeben.

Nach Freigabe können im Betrieb die im RFID-Tag gespeicherten Daten, zum Beispiel in Form von Parametern für eine Ablaufsteuerung und/oder für eine Berechnung beispielsweise des Blutglukosewertes verwendet werden. Man kann sagen, dass eine individuelle Parametrierung der Berechnung auf die in der Produktion ermittelten Chargencharakterisierungswerte des Einmalartikels erfolgen kann.

Ein besonderer Vorteil der Erfindung ist die sichere, zusätzliche Möglichkeit, dass Daten im RFID-Tag während des Betriebs modifiziert werden können und dabei sichergestellt ist, dass keine Daten verfälscht werden oder nur teilweise korrekt überschrieben werden.

Vorteilhafte Ausführungsformen der Erfindung sind in den Unteransprüchen beansprucht und werden nachfolgend näher erläutert.

Eine bevorzugte Ausführungsform der Erfindung, welche ggf. unabhängig zu beanspruchen ist, sieht vor, die Daten (Parameter) nach dem Prinzip der "iterativen doppelten Speicherung mit jeweiliger (individuell zugeordneter, zueinander unterschiedlicher) Prüfsumme" auf den RFID-Tag abzulegen. Demnach ist das bevorzugte Speicherverfahren dadurch gekennzeichnet, dass die Daten in Form eines ersten (alten/ursprünglichen, bzw. unmittelbar vorhergehenden) Datensatzes und eines zweiten (neuen, bzw. unmittelbar nachfolgenden) Datensatzes auf dem RFID-Tag gespeichert (geschrieben) werden und jeder der beiden Datensätze mit einer eigenen (zueinander unterschiedlichen) Prüfsumme (Code) versehen wird. Der erste (alte) Datensatz enthält somit Daten der ersten bzw. aktuellen Version und der zweite (neue) Datensatz enthält überarbeitete (zukünftige) Daten, der zweiten, bzw. von nun an geltenden Version.

Dies ist besonders vorteilhaft in einem Fall, in dem der Einmalartikel von einem Anwender zu einem beliebigen Zeitpunkt vom wiederverwendbaren Teil des Messsystems entfernt wurde. Dies kann nämlich unter Umständen insbesondere zu einem Zeitpunkt erfolgen, zu dem gerade ein Schreibvorgang in den RFID-Tag erfolgt, aber noch nicht abgeschlossen ist. Der Schreibvorgang wäre dann unvollständig, was zu korrumpierten/falschen Daten im RFID-Speicher führen könnte, da teils alte, teils neue Daten vorhanden wären. Im Fall einer derart unvollständigen Datenüberschreibung im zweiten (neuen) Datensatz stimmt die daraus resultierende Prüfsumme nicht mit der theoretisch zu erwartenden Prüfsumme überein, woraus das Steuermodul auf einen falschen/unvollständig überschriebenen zweiten Datensatz schließt und daher auf den ersten (alten) Datensatz als "fall-back"-Datensatz zurückgreifen kann.

Durch die vorstehende Ausführungsform des Verfahrens kann in besonders vorteilhafter Weise sichergestellt werden, dass stets zumindest ein Satz korrekter (wenngleich ggf. nicht auf den allerletzten Stand up-gedateter) Daten auf dem RFID-Tag vorhanden ist, auf den notfalls zurückgegriffen werden kann. Durch den Notfall-Rückgriff auf die Daten der aktuellen (zuletzt up-gedateten) ersten Version bei fehlerhafter Erstellung der neuen, zweiten Version ist kein Behandlungsrisiko zu befürchten. Es wird somit ein Verfahren bereitgestellt, mit dem die Funktion des jederzeit im Betrieb vom Anwender abnehmbaren Einmalartikels (Sensors) infolge des Ausschlusses verfälschter/unvollständiger Datensätze gesichert werden kann. Auf diese Weise kann ein Abnehmen des Einmalartikels (Sensors) vom Messsystem durch den Anwender nicht zu korrumpierten Daten führen, die einen späteren Weiterbetrieb be- oder verhindern.

Die (aktuell geltenden) Daten/Datensätze können insbesondere zyklisch und/oder ereignisgesteuert modifiziert werden und dabei in den RFID-Tag geschrieben werden, wobei jedes Mal der bis dahin geltende Datensatz zum "fall-back"-Datensatz wird und der neue/modifizierte Datensatz im Fall seiner Unverfälschtheit zum aktuell (von nun an) geltenden Datensatz wird. Vorzugsweise wird also bei einer Modifizierung von Daten quasi nur jeweils einer der beiden Datensätze modifiziert und der andere der beiden Datensätze verbleibt unverändert. Die Daten können insbesondere mittels der Software durch einen RFID-Writer/Reader geschrieben werden, der im wiederverwendbaren Teil hinterlegt ist.

Alternativ oder zusätzlich kann im Falle einer negativen Plausibilitätsprüfung eines der beiden Datensätze der andere der beiden Datensätze einer Plausibilitätsprüfung unterzogen werden. Ist diese positiv, können die Daten des negativ geprüften Datensatzes mit denen des positiv geprüften Datensatzes repariert werden, was im einfachsten Fall durch Überschreiben mit den Daten des positiv geprüften Datensatzes erfolgt. Nachfolgend kann dann der neuere der beiden dann sicher nicht korrumpierten Datensätze für eine weitere Durchführung des Verfahrens genutzt werden. Welcher der beiden Datensätze neuer ist, wird, sofern nicht die Datensätze selbst einen Zählerwert darstellen, durch je einen im jeweiligen Datensatz zusätzlich enthaltenen Zählerwert festgestellt.

Eine weitere Ausführungsform der Erfindung ist dadurch gekennzeichnet, dass Schreibzugriffe wechselweise auf einen der beiden Datensätze ausgeführt werden. Erst wenn ein Schreibzugriff beendet ist und die Prüfsumme des beschriebenen Datensatzes fehlerfrei ist, kann ein Zugriff auf den anderen Datensatz erfolgen. Im Falle einer Beschädigung des beschriebenen Datensatzes kann der unbeschriebene (nicht modifizierte) Datensatz zu dessen Reparatur und/oder zur weiteren Durchführung des Verfahrens herangezogen werden. Auf diese Weise steht stets ein nicht korrumpierter Datensatz zur Durchführung des Verfahrens zur Verfügung.

Es ist besonders vorteilhaft, wenn bei einem erstmaligen Auslesen von Daten nach einem Verbinden des Einmalartikels mit dem Messsystem beide Datensätze ausgelesen und einer Plausibilitätsprüfung unterzogen werden. So wird sichergestellt, dass im Falle einer Beschädigung eines der Datensätze beim Beschreiben auf den anderen Datensatz mit nicht korrumpierten Daten zurückgegriffen werden kann.

Eine Ausführungsform der Erfindung ist dadurch gekennzeichnet, dass beide Datensätze einer Plausibilitätsprüfung unterzogen werden und in einem Fall, in dem beide Plausibilitätsprüfungen positiv sind, der jüngere der beiden Datensätze verwendet wird. Auf diese Weise werden zur Durchführung des Verfahrens stets möglichst aktuelle Daten verwendet. Die Daten des älteren Datensatzes können in einem solchen Fall, auch wenn sie nicht beschädigt sind, mit den positiv geprüften Daten des jüngeren Datensatzes überschrieben werden.

Die Daten können nach der Erfindung insbesondere vollständig oder teilweise verschlüsselt werden. Auf diese Weise ist der Inhalt der Daten der Speicherzellen oder des RFID-Tags nur mehr mit Hilfe eines zusätzlich erforderlichen Schlüssels zu interpretieren. Für die Verschlüsselung kann grundsätzlich ein beliebiger Algorithmus verwendet werden. Als Schlüssel kann die in dem RFID-Tag herstellerseitig enthaltene feste, unveränderliche eindeutige Identifikationsnummer, auch als UID (Unique Identifier) bezeichnet, dienen. Die eindeutige Identifikationsnummer des RFID-Tags kann bereits herstellerseitig fester, unveränderlicher Bestandteil des RFID-Tags sein und braucht daher nicht gesondert geschrieben zu werden. Durch eine Verschlüsselung unter Verwendung der UID kann ein erhöhter Schutz gegen unautorisierte Nachbauten erreicht werden.

Der Einmalartikel kann insbesondere im Rahmen der Herstellung einer Sterilisation unterzogen werden. Dabei kann er mit integriertem RFID-Tag verpackt und sterilisiert werden, insbesondere mittels einer Strahlensterilisation. Eine Besonderheit ist, dass der RFID-Tag die Belastung der Strahlensterilisation aushält, ohne zerstört zu werden oder den Dateninhalt zu verlieren. Erreicht werden kann dies durch Auswahl eines besonderen RFID-Tags auf Basis eines FRAM-Speichers anstelle eines üblichen EEPROM-Speichers.

Die Daten des auf dem RFID-Tag enthaltenen Datensatzes können zumindest ein Element/mehrere Elemente der Gruppe
- Version,
- Herstellungsdatum,
- Verwendungsdatum,
- Produktionsanlagenkennung,
- chargenspezifische Kenndaten und
- applikationsspezifische Kenndaten
   betreffen. Sie können alle oder teilweise einer Plausibilitätsprüfung des Einmalartikels und/oder Überwachung des Einmalartikels und/oder einer Parametrierung von Softwarealgorithmen (z.B. Messalgorithmen) dienen. Des Weiteren können die in dem RFID-Tag gespeicherten Daten für eine Steuerung der Messung und/oder für eine Berechnung des zu messenden Blutwerts verwendet werden. Solche Daten betreffen insbesondere Parameter wie
- eine Applikationsdauer des Einmalartikels und/oder
- eine Anzahl von Messungen und/oder
- eine Blutkontaktdauer und/oder
- eine Belichtungsdosis.
Nach Erreichen einer festgelegten Bedingung für diese Parameter, insbesondere nach Erreichen einer maximalen Applikationsdauer und/oder einer maximalen Anzahl von Messungen und/oder einer maximal erlaubten Blutkontaktdauer und/oder einer maximalen Belichtungsdosis, kann ein Weiterbetrieb im Wesentlichen verhindert bzw. blockiert werden. Ggf. kann eine Fehlermeldung ausgegeben werden.

Durch die Erfindung können insbesondere die Vorteile erzielt werden, dass der Einmalartikel (zum Beispiel ein Sensor) mit einem Messsystem, an das er angeschlossen wird, automatisiert, d.h. ohne eine Aktion des Anwenders, Daten austauschen kann. Das Messsystem kann stets mit den korrekten Daten spezifisch für diesen Einmalartikel arbeiten. Eine Verwendung des Einmalartikels über seine bestimmungsgemäße Verwendung hinaus (z.B. maximale Applikationsdauer) kann im Wesentlichen (d.h. ggf. vernachlässigbares Überschreiten) verhindert werden. Der Anwender kann zudem den Einmalartikel während des Betriebs jederzeit vom Messsystem abnehmen und wieder anbringen, ohne dass der Weiterbetrieb aufgrund korrumpierter Daten verhindert wird.

Zusammenfassend kann man auch sagen, dass die Erfindung eine Vorrichtung und ein Verfahren unter Verwendung von RFID-Technik ermöglicht bzw. bereitstellt, wobei automatisiert Daten eines abnehmbaren Einmalartikels, zum Beispiel in Form eines Einmalsensors, mit einem wiederverwendbaren Teil eines physiologischen Messsystems auszutauschen sind. Die Daten (sowohl konstante, als auch sensorspezifisch veränderliche Daten) werden bei der Herstellung des Einmalartikels in den Speicher eines RFID-Tags geschrieben und während dessen Verwendung gelesen, ggf. (zyklisch oder anwendungsspezifisch) modifiziert und wieder in den Speicher geschrieben. Die Daten können insbesondere einer Plausibilitätsprüfung und/oder Überwachung des Einmalartikels und der Parametrierung von Softwarealgorithmen (z.B. Messalgorithmen) dienen.

Insbesondere kann das Verfahren einen oder mehrere der folgenden Schritte aufweisen:
a) Der Sensor (als Einmalartikel) erhält im Rahmen einer Assemblierung einen RFID- Tag, der in einem Gehäuse des Sensors montiert wird.
b) Der RFID- Tag wird mit Daten beschrieben, die sowohl konstante, als auch individuelle und chargenspezifische Daten sein können. Als Schutz gegen unerkannte Verfälschung von Daten werden diese mit einer oder mehreren Prüfsummen (aus dem Stand der Technik bekanntes Verfahren) versehen.
c) Um gegen einfaches Kopieren des Speicherinhalts auf einen anderen RFID-Tag geschützt zu sein, wird zusätzlich eine Signatur (ebenfalls aus dem Stand der Technik bekannte Verschlüsselung) gespeichert, die sich vorzugsweise aus dem individuellen Speicherinhalt errechnet (Kopierschutz).
d) Der Sensor mit integriertem RFID-Tag wird verpackt und sterilisiert, insbesondere mittels einer Strahlensterilisation.
e) Bei der Verwendung des Sensors liest ein im Messsystem bzw. dessen im wiederverwendbaren Teil befindlicher RFID-Reader/Writer den Speicherinhalt des RFID-Tags aus, sobald der Sensor auf den wiederverwendbaren Teil des Messsystems aufgesteckt/gekoppelt wird, und überträgt ihn an ein Steuerungsmodul (im wiederverwendbaren Teil des Messsystems).
f) Eine Software im Steuerungsmodul führt Plausibilitätsprüfungen über den Speicherinhalt durch und gibt bei positivem Prüfergebnis den Sensor zur Verwendung frei.
g) Im Betrieb verwendet die Software im Steuerungsmodul die im RFID- Tag gespeicherten Parameter vorzugsweise für die Ablaufsteuerung und/oder die Berechnung des Blutglukosewertes (individuelle Parametrierung der Berechnung auf die in der Produktion ermittelten Chargencharakterisierungswerte des Einmalartikels).
h) Im Betrieb werden vorzugsweise zyklisch Parameter in den RFID-Tag geschrieben (wie z.B. Applikationsdauer, Blutkontaktdauer, Lichtdosis der chemischen Spots). Diese Werte dienen zur Überwachung der Messbedingungen.
i) Im Falle einer Entfernung oder Entkopplung des Sensors durch einen Anwender zu einem (ungünstigen) Zeitpunkt vom Messsystem, insbesondere zu einem Zeitpunkt, an dem ein gerade erfolgender Schreibvorgang in den RFID-Tag unterbrochen wird, was zu korrumpierten Daten im RFID-Speicher führen kann, wird ein besonderes Verfahren (nämlich das iterative doppelte Speicherverfahren mit Prüfsumme gemäß vorstehender Definition) mit zumindest zwei separaten Datensätzen angewandt, um stets einen Satz korrekter Daten zu haben.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
Fig. 1 einen Datensatz, wie er in einem bekannten Verfahren nach dem Stand der Technik verwendet wird,
Fig. 2 den Datensatz der Figur 1 im Falle einer Korrumpierung von Daten durch Trennen von Einmalartikel und Messsystem,
Fig. 3 einen Datensatz, wie er bei einer Ausführungsform der Erfindung genutzt wird.

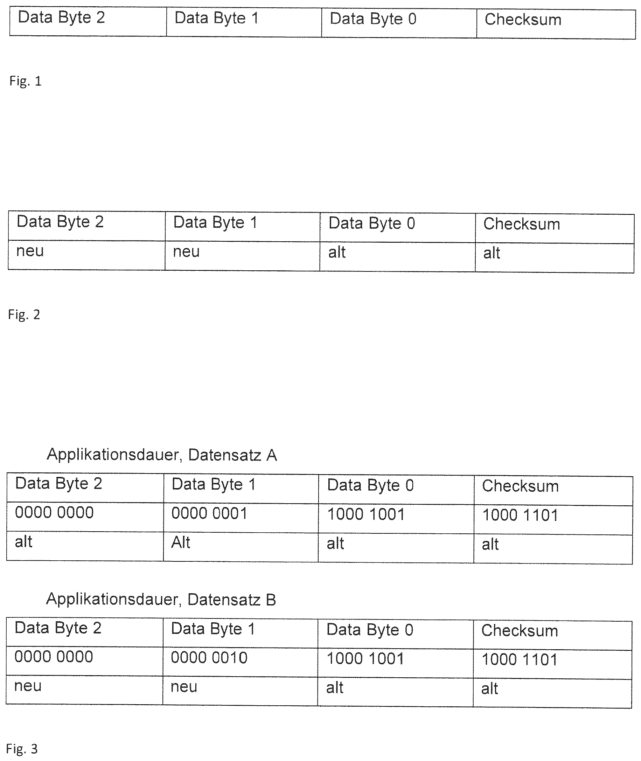

Eine Verwendung einer Prüfsumme ist ein bekanntes Verfahren, um Daten gegen unerkannte Verfälschung zu sichern, und ist zum Beispiel in der Norm EN 61508-7: 2010, A/1,2 beschrieben. Man kann sagen, dass dabei Daten in Datenblöcke unterteilt werden und ein gesonderter Datenblock aus diesen als Prüfsumme erstellt wird. Das Beispiel der Figur 1 zeigt das Prinzip bei Daten mit drei Byte Länge und einem Byte Prüfsumme, hier als "Checksum" bezeichnet. Die Prüfsumme errechnet sich dabei mit Hilfe eines hier nicht näher bestimmten Algorithmus aus den Daten der vorstehenden drei Datenbytes.

Sollen im Betrieb Daten in den Speicher des RFID-Tag geschrieben werden oder darin bereits hinterlegte Daten modifiziert, also überschrieben, werden, weil zum Beispiel die Applikationsdauer oder die Anzahl der durchgeführten Messungen zu aktualisieren sind, erfolgt der Schreibzugriff nach bekannten Verfahren partiell. Das bedeutet, dass nur ein Byte nach dem anderen oder nur Gruppen von zum Beispiel zwei Bytes gleichzeitig modifiziert und überschrieben werden können. In der Folge kann es bei bekannten Verfahren vorkommen, dass, wenn ein Anwender während eines solchen Schreibvorgangs den Einmalartikel vom Messsystem entkoppelt, ein Teil der Daten bereits mit neuem Inhalt beschrieben wurde und ein anderer Teil noch den alten Inhalt enthält. Das Beispiel der Figur 2 verdeutlicht diese Situation. In einem solchen Fall sind bei einem bekannten Verfahren die auf dem RFID-Tag vorliegenden Daten korrumpiert, d.h. der tatsächliche Inhalt entspricht nicht dem gewollten Inhalt. Die Prüfung der "Checksum" wird eine solche Datenverfälschung zwar erkennen, jedoch sind die Daten für einen späteren Weiterbetrieb des Einmalartikels nicht mehr verwendbar.

Das Verfahren nach der vorliegenden Ausführungsform bietet in diesem Fall eine Lösung, die in einer iterativen doppelten Speicherung der Daten jeweils mit Prüfsumme besteht. Jeder Parameter, der im Betrieb modifiziert wird, also geschrieben/überschrieben und später wieder gelesen werden muss, wird zweimal, also doppelt, gespeichert, jeweils mit eigener Prüfsumme. Das Schreiben erfolgt zyklisch und/oder ereignisgesteuert. Der eigentliche Schreibvorgang läuft identisch ab wie vorstehend beschrieben, jedoch wird nur einer der beiden Datensätze modifiziert und überschrieben, während der andere Datensatz unangetastet bleibt und so nicht modifiziert wird. Dadurch ist sichergestellt, dass im Fall von korrumpierten Daten jeweils ein intakter, unverfälschter Datensatz des jeweiligen Parameters existiert, mit dessen Inhalt ein Weiterbetrieb möglich ist. Das Beispiel der Figur 3 verdeutlicht der Datensatz A des Parameters der Applikationsdauer den ersten Datensatz und der Datensatz B des Parameters der Applikationsdauer den zweiten Datensatz. In diesem Beispiel wurde angenommen, dass der Einmalartikel nach einem Schreibzugriff auf Byte 2 und Byte 1 des Datensatzes B entfernt wurde, bevor die noch ausstehenden Schreibzugriffe auf Byte 0 und die Checksum ausgeführt werden konnten. Bei einem erneuten Verbinden des Einmalartikels mit dem Messsystem kann nun bei dieser Ausführungsform des erfindungsgemäßen Verfahrens die Prüfsummenprüfung hinsichtlich des Datensatzes B die korrumpierten Daten feststellen und einen Weiterbetrieb dieses Einmalartikels verhindern. Da aber noch ein zweiter, unverfälschter Datensatz dieses Parameters existiert, nämlich der Datensatz A, kann auf diesen zurückgegriffen werden und der Weiterbetrieb mit diesem zweiten Datensatz ermöglicht werden.

Ein beispielhaftes Verfahren nach der Erfindung verwendet die folgenden Schritte:
- Jeder Parameter, der im Betrieb modifiziert, d.h. geschrieben und später wieder gelesen werden muss, wird zweimal gespeichert mit jeweils eigener Prüfsumme
- Schreibzugriffe werden wechselweise auf den beiden Datensätzen eines Parameters ausgeführt.
- Ein Schreibzugriff auf einen Datensatz erfolgt nur, wenn der andere Datensatz nicht korrumpiert ist.
- Beim Verbinden des Einmalartikels mit dem Messsystem werden beide Datensätze eines Parameters gelesen.
- Die Entscheidung, welcher Parameter verwendet wird, ergibt sich nach folgender Logik:
- Ergibt die Prüfsummenprüfung für beide Datensätze eine Verfälschung, so kann keiner der beiden verwendet werden (dieser Fall sollte im Normalfall nicht vorkommen).
- Ergibt die Prüfsummenprüfung für einen Datensatz eine Verfälschung, so wird der andere verwendet (dieser Fall kann bei einem zuvor unterbrochenen Schreibvorgang vorkommen). Dieser andere, nicht korrumpierte Datensatz dient anschließend der Reparatur des korrumpierten Datensatzes.
- Ergibt die Prüfsummenprüfung für beide Datensatze die Korrektheit, so wird derjenige verwendet, dessen Inhalt neuer ist, was z.B. bei einem dekrementierenden Zähler eindeutig feststellbar ist (dieser Fall kann vorkommen, wenn zuvor alle Schreibvorgänge vollständig ausgeführt werden konnten).
- Auf diese Weise ist sichergestellt, dass immer ein verwendbarer Datensatz zur Verfügung steht. Die Unschärfe bzgl. der Aktualität des Inhalts kann durch ein geeignetes Updateintervall hinreichend klein gehalten werden.

## Patentansprüche

1. Verfahren zum Austausch von Daten eines Einmalartikels, vorzugsweise Sensors, mit einem wiederverwendbaren Teil, vorzugsweise Steuermodul, eines medizintechnischen Messsystems bei einer Messung eines Blutwerts, insbesondere eines Blutglukosewerts, wobei die Daten, die konstante, individuelle oder chargenspezifische Daten sind, für eine Steuerung der Messung und/oder für eine Berechnung des zu messenden Blutwerts verwendet werden und für eine Überwachung der Messbedingungen dienen, wonach
diese Daten bei einer Herstellung des Einmalartikels in einen Speicher eines RFID-Tags des Einmalartikels geschrieben werden und zumindest eine Prüfsumme aufweisen bzw. mit einer zugeordneten Prüfsumme versehen sind,
der Einmalartikel mit dem Messsystem zumindest datentechnisch und vorzugsweise auch mechanisch verbunden wird,
die im RFID-Tag enthaltenen Daten vom wiederverwendbaren Teil des Messsystems ausgelesen und einer Plausibilitätsprüfung anhand der zumindest einen Prüfsumme unterzogen werden,
bei positiver Plausibilitätsprüfung der Einmalartikel zur Verwendung im Messsystem freigegeben wird, und
die Daten nach dem Prinzip der iterativen, doppelten Speicherung mit Prüfsumme auf dem RFID-Tag abgespeichert werden, bei welchem die Daten in Form eines ersten Datensatzes und eines zweiten Datensatzes auf dem RFID-Tag gespeichert werden und jeder der beiden Datensätze mit einer eigenen Prüfsumme versehen wird, wobei der erste Datensatz Daten der ersten Version und der zweite Datensatz überarbeitete Daten hat, wobei im Fall einer unvollständigen Datenüberschreibung im zweiten Datensatz die resultierende Prüfsumme nicht mit der theoretisch zu erwartenden Prüfsumme übereinstimmt und das wiederverwendbare Teil auf den ersten Datensatz zurückgreift.

2. Verfahren nach Anspruch 1, **dadurch gekennzeichnet, dass** die Daten zyklisch und/oder ereignisgesteuert modifiziert werden und dabei in den RFID-Tag geschrieben werden.

3. Verfahren nach einem der vorstehenden Ansprüche, **dadurch gekennzeichnet, dass** bei einer Modifizierung von Daten nur jeweils einer der beiden Datensätze modifiziert wird und der andere der beiden Datensätze unverändert verbleibt.

4. Verfahren nach einem der vorstehenden Ansprüche, **dadurch gekennzeichnet, dass** im Falle einer negativen Plausibilitätsprüfung eines der beiden Datensätze der andere der beiden Datensätze einer Plausibilitätsprüfung unterzogen wird.

5. Verfahren nach einem der vorstehenden Ansprüche, **dadurch gekennzeichnet, dass** im Falle einer positiven Plausibilitätsprüfung eines der beiden Datensätze der andere der beiden Datensätze mit den positiv geprüften Daten des einen Datensatzes überschrieben wird.

6. Verfahren nach einem der vorstehenden Ansprüche, **dadurch gekennzeichnet, dass** Schreibzugriffe wechselweise auf einen der beiden Datensätze ausgeführt werden.

7. Verfahren nach einem der vorstehenden Ansprüche, **dadurch gekennzeichnet, dass** bei einem erstmaligen Auslesen von Daten nach einem datentechnischen und vorzugsweise mechanischen Verbinden des Einmalartikels mit dem Messsystem beide Datensätze ausgelesen und einer Plausibilitätsprüfung unterzogen werden.

8. Verfahren nach einem der vorstehenden Ansprüche, **dadurch gekennzeichnet, dass** beide Datensätze einer Plausibilitätsprüfung unterzogen werden und in einem Fall, in dem beide Plausibilitätsprüfungen positiv sind, der jüngere der beiden Datensätze verwendet wird.

9. Verfahren nach einem der vorhergehenden Ansprüche, **dadurch gekennzeichnet, dass** die Daten vollständig oder teilweise verschlüsselt werden.

## Claims

1. Method for interchanging data of a disposable item, preferably a sensor, with a reusable part, preferably a control module, of a medical measurement system during a measurement of a blood value, in particular a blood glucose value, wherein the data, which are constant, individual, or batch-specific data, are used for controlling the measurement and/or for a calculation of the blood value to be measured and serve for monitoring the measurement conditions, according to which
these data are written into a memory of an RFID tag of the disposable item during production of the disposable item and have at least one checksum or, respectively, are provided with an assigned checksum,
the disposable item is connected to the measurement system at least in a data-related manner and preferably also mechanically,
the data contained in the RFID tag are read out by the reusable part of the measurement system and subjected to a plausibility check on the basis of the at least one checksum,
if the plausibility check is positive, the disposable item is released for use in the measurement system, and
the data are stored on the RFID tag according to the principle of iterative double storage with checksum, wherein the data are stored on the RFID tag in the form of a first data set and a second data set and each of the two data sets is provided with its own checksum, wherein the first data set has data of the first version and the second data set has revised data, wherein in the case of an incomplete data overwrite in the second data set, the resulting checksum does not correspond to the theoretically expected checksum and the reusable part accesses the first data set.

2. The method according to claim 1, **characterized in that** the data is modified cyclically and/or in an event-driven manner and is thereby written to the RFID tag.

3. The method according to one of the preceding claims, **characterized in that,** in the case of a modification of data, only one of the two data sets is modified in each case and the other one of the two data sets remains unchanged.

4. The method according to one of the preceding claims, **characterized in that** in case of a negative plausibility check of one of the two data sets, the other one of the two data sets is subjected to a plausibility check.

5. The method according to one of the preceding claims, **characterized in that** in case of a positive plausibility check of one of the two data sets, the other one of the two data sets is overwritten with the positively checked data of the one data set.

6. The method according to one of the preceding claims, **characterized in that** write accesses are performed alternately on one of the two data sets.

7. The method according to one of the preceding claims, **characterized in that** during an initial reading of data after a data-related and preferably mechanical connection of the disposable item to the measurement system, both data sets are read out and subjected to a plausibility check.

8. The method according to one of the preceding claims, **characterized in that** both data sets are subjected to a plausibility check and in a case in which both plausibility checks are positive, the more recent of the two data sets is used.

9. The method according to one of the preceding claims, **characterized in that** the data are fully or partially encrypted.

## Revendications

1. Procédé pour l'échange de données d'un article jetable, de préférence capteur, avec une partie réutilisable, de préférence un module de commande, d'un système de mesure à usage médical lors d'une mesure d'une valeur sanguine, en particulier d'une valeur de glycémie, dans lequel les données, qui sont des données constantes, individuelles ou spécifiques à un lot, sont utilisées pour une commande de la mesure et/ou pour un calcul de la valeur sanguine à mesurer et servent à une surveillance des conditions de mesure, après quoi
ces données sont inscrites dans une mémoire d'une étiquette RFID de l'article jetable lors d'une fabrication de l'article jetable et présentent au moins une somme de contrôle ou sont pourvues d'une somme de contrôle associée,
l'article jetable est relié au système de mesure au moins par technique informatique et de préférence également mécaniquement,
les données contenues dans l'étiquette RFID sont lues par la partie réutilisable du système de mesure et soumises à un contrôle de plausibilité sur la base de la au moins une somme de contrôle,
lorsque le contrôle de plausibilité est positif l'article jetable est libéré pour être utilisé dans le système de mesure, et
les données sont mises en mémoire sur l'étiquette RFID selon le principe de la mise en mémoire double, itérative avec la somme de contrôle, selon lequel les données sont mises en mémoire sur l'étiquette RFID sous forme d'un premier ensemble de données et d'un deuxième ensemble de données et chacun des deux ensembles de données est pourvu d'une somme de contrôle propre, dans lequel le premier ensemble de données possède des données de la première version et le deuxième ensemble de données des données corrigées, dans lequel dans le cas d'un écrasement de données incomplet dans le deuxième ensemble de données la somme de contrôle résultante ne coïncide pas avec la somme de contrôle théoriquement attendue et la partie réutilisable recourt au premier ensemble de données.

2. Procédé selon la revendication 1, **caractérisé en ce que** les données sont modifiées de manière cyclique et/ou de manière commandée par l'événement et ce faisant sont inscrites dans l'étiquette RFID.

3. Procédé selon l'une quelconque des revendications précédentes, **caractérisé en ce que** lors d'une modification de données seule respectivement un des deux ensembles de données est modifié et l'autre des deux ensembles de données reste inchangé.

4. Procédé selon l'une quelconque des revendications précédentes, **caractérisé en ce que** dans le cas d'un contrôle de plausibilité négatif d'un des deux ensembles de données l'autre des deux ensembles de données est soumis à un contrôle de plausibilité.

5. Procédé selon l'une quelconque des revendications précédentes, **caractérisé en ce que** dans le cas d'un contrôle de plausibilité positif d'un des deux ensembles de données l'autre des deux ensembles de données est écrasé avec les données contrôlées de manière positive de l'un des ensembles de données.

6. Procédé selon l'une quelconque des revendications précédentes, **caractérisé en ce que** des accès en écriture sont réalisés alternativement sur un des deux ensembles de données.

7. Procédé selon l'une quelconque des revendications précédentes, **caractérisé en ce que** lors de la première lecture de données après une liaison par technique informatique et de préférence mécanique de l'article jetable au système de mesure les deux ensembles de données sont lus et soumis à un contrôle de plausibilité.

8. Procédé selon l'une quelconque des revendications précédentes, **caractérisé en ce que** les deux ensembles de données sont soumis à un contrôle de plausibilité et dans un cas dans lequel les deux contrôles de plausibilité sont positifs, le plus récent des deux ensembles de données est utilisé.

9. Procédé selon l'une quelconque des revendications précédentes, **caractérisé en ce que** les données sont entièrement ou partiellement chiffrées.
